# EUROPÄISCHE PATENTSCHRIFT

(11) **EP 3 320 969 B1**
(45) Veröffentlichungstag und Bekanntmachung des Hinweises auf die Patenterteilung: **20.05.2020**
(21) Anmeldenummer: 17200457.4
(22) Anmeldetag: 07.11.2017
(51) Int. Cl.: B01J 21/08, B01J 23/72, C07C 29/141, C07C 31/125

(54) **CHROM- UND NICKELFREIE HYDRIERUNG VON HYDROFORMYLIERUNGSGEMISCHEN**
CHROMIUM AND NICKEL-FREE HYDROGENATION OF HYDROFORMYLATION MIXTURES
HYDROGÉNATION SANS NICKEL NI CHROME DE MÉLANGES D'HYDROFORMLYLATION

(30) Priorität: 09.11.2016 EP 16197935
(43) Veröffentlichungstag der Anmeldung: 16.05.2018
(73) Patentinhaber: Evonik Operations GmbH, 45128 Essen (DE)
(72) Erfinder: Klasovsky, Florian, 45721 Haltern am See (DE); Franke, Robert, 45772 Marl (DE); Geilen, Frank, 45721 Haltern am See (DE); Jess, Andreas, 95447 Bayreuth (DE); Korth, Wolfgang, 95447 Bayreuth (DE); Quandt, Thomas, 45772 Marl (DE); Reinsdorf, Arne, 64283 Darmstadt (DE)
(74) Vertreter: Evonik Patent Association

(56) Entgegenhaltungen:
- WO-A1-95/32171
- US-A- 4 677 234

## Beschreibung

Die Erfindung betrifft ein Verfahren zur Herstellung von Alkoholen durch Hydrierung von Aldehyden, bei welchem ein Einsatzgemisch enthaltend mindestens einen Aldehyd sowie mindestens eine Begleitkomponente in Gegenwart von Wasserstoff mit einem heterogenen Katalysator in Kontakt gebracht wird, wodurch ein Produktgemisch erhalten wird, welches zumindest den dem hydrierten Aldehyd entsprechenden Alkohol sowie zumindest ein Nebenprodukt enthält, wobei der Katalysator ein Trägermaterial sowie darauf aufgebracht Kupfer umfasst.

Des Weiteren betrifft die Erfindung die Herstellung des zugehörigen Katalysators bzw. des zugehörigen Präkursors, ggf. Aktivierung des Präkursors und Verwendung des aktiven Katalysators in dem Verfahren.

Durch Abspaltung von Wasserstoff (Dehydrierung) von einem Alkohol entsteht ein Aldehyd. Umgekehrt lassen sich Alkohole durch Hydrierung (Anlagerung von Wasserstoff) von Aldehyden herstellen.

Die Hydrierung im Allgemeinen ist eine in der industriellen Technik sehr häufig durchgeführte Reaktion. Aber auch speziell die Hydrierung von Aldehyden wird großindustriell praktiziert, nämlich bei der Herstellung von so genannten Oxo-Alkoholen.

Oxo-Alkohole sind Alkohole, die im Wege der Hydroformylierung (Oxo-Reaktion) hergestellt werden. Bei der Hydroformylierung wird ein Olefin (Alken) mit Synthesegas (das ist eine Mischung aus Kohlenmonoxid und Wasserstoff) zu einem Aldehyd umgesetzt. Durch anschließende Hydrierung wird der eigentliche Oxo-Alkohol erhalten. Oxo-Alkohole dienen als Zwischenprodukte für die Herstellung von Tensiden bzw. Weichmachern für Kunststoff. Weltweit werden jährlich mehrere Millionen Tonnen Oxo-Alkohole hergestellt.

Da die Hydrierung der durch die Hydroformylierung erhaltenen Aldehyde ein notwendiger Schritt bei der Herstellung von Oxo-Alkoholen ist, beschäftigt sich die vorliegende Erfindung mit einem großindustriell relevanten Prozess.

In der industriellen Praxis werden Oxo-Aldehyde in der Regel in der Flüssigphase an heterogenen Festbett-Katalysatoren hydriert. Aufgrund der großen Durchsatzmengen kommt dem Katalysator die entscheidende Bedeutung für den Prozess zu, da er die Reaktionsgeschwindigkeit und auch die Selektivität der Hydrierung bestimmt. Die Auswahl eines geeigneten Katalysators ist nicht trivial, da die zu hydrierenden Aldehyde niemals rein vorkommen, sondern als ein Gemisch von strukturisomeren Aldehyden, welches stets von einer großen Anzahl störender Begleitkomponenten begleitet wird, die zum einen in der Hydrierung unerwünschte Nebenreaktionen hervorrufen und zum anderen den Hydrierkatalysator schädigen. Da die Zusammensetzung des die zu hydrierenden Aldehyde enthaltenden Einsatzgemisches durch die vorgelagerte Hydroformylierung bestimmt wird, ist der Hydrierkatalysator auf die jeweilige Hydroformylierung genau anzupassen.

Bei der Hydrierung von Oxo-Aldehyden haben sich solche Katalysatoren bewährt, die ein Trägermaterial umfassen, auf welchem als aktive Komponenten Kupfer, Chrom und Nickel aufgebracht sind.

Ein entsprechender Katalysator ist in DE19842370A1 offenbart. Er umfasst Kupfer und Nickel jeweils in einem Konzentrationsbereich von 0.3 bis 15 Gew.-% und Chrom zu einem Gewichtsanteil von 0.05 Gew.-% bis 3.5 Gew.-%. Als Trägermaterial wird poröses Siliciumdioxid oder Aluminiumoxid verwendet.

US 4677234 beschreibt ein Verfahren zur Herstellung von Ethylenglycol in Gegenwart eines trägergestützten Kupferkatalysators.

Wenngleich diese Katalysatoren sich in der industriell praktizierten Hydrierung von Oxo-Aldehyden bewährt haben, bestand doch das Bedürfnis nach einer Alternative. Grund dafür ist der ChromGehalt dieser Katalysatoren.

Gemäß Annex XIV der REACH-Verordnung dürfen chromhaltige Substanzen wie die vorbeschriebenen Katalysatoren in der Europäischen Union nur noch nach Autorisierung durch die Kommission verwendet werden. Die Erteilung der Autorisierung ist mit hohem Aufwand und Kosten verbunden; zudem kann nicht a priori mit der Erteilung der Autorisierung gerechnet werden. Die Anmeldeprozedur muss darüber hinaus alle fünf Jahre wiederholt werden.

Grund für diese strengen Auflagen ist die unbestrittene Kanzerogenität des verwendeten Chroms. Diese ist zum einen dann relevant, wenn der Hydrierkatalysator nach Desaktivierung entsorgt werden muss und zum anderen wenn er durch Imprägnierung mit Alkalichromaten oder Alkalidichromate neu hergestellt wird.

Das Chrom-Problem wurde gelöst mit dem in EP3037400A1 offenbarten Katalysator, welcher nahezu chromfrei ist. Allerdings besteht bei diesem System weiterer Verbesserungsbedarf, da Nickel und die bei der Herstellung des chromfreien Katalysators verwendeten Ni-Verbindungen ebenfalls kanzerogen sind.

Insoweit besteht die Aufgabe, ein zur industriellen Hydrierung von Aldehyden geeignetes Katalysatorsystem anzugeben, welches sowohl frei von Chrom, als auch frei von Nickel ist.

EP2488478B1 beschreibt eine zweitstufige Hydrierung von C₁₀-Aldehyden, wobei in der zweiten Hydrierstufe ein Katalysator eingesetzt wird, welcher frei von Kupfer, Chrom und Nickel ist, dafür aber Ruthenium enthält. Ru ist vergleichsweise teuer, weswegen dieser Prozess im industriellen Maßstab nicht immer wirtschaftlich ist. Darüber ist der Prozess auch nicht nickelfrei, da in der ersten Stufe ein Nickel-haltiger Katalysator eingesetzt werden muss, um akzeptable Hydrierergebnisse zu erzielen.

WO95/32171A1 beschreibt diverse Hydrierkatalysatoren enthaltend Kupfer und Siliciumdixoid wahlweise in Anwesenheit oder Abwesenheit weitere Elemente wie unter anderem Chrom. Die konkreten, chromfreien Varianten fallen durch sehr hohe CuO-Anteile (weit über 20 Gew.-%) auf. Die Rohstoffkosten für solch kupferreichen Katalysatoren sind recht hoch.

In US 3,677,969 wird ein metallorganischer Hydrierkatalysator beschrieben. Nachteil dieses Systems ist, dass seine Herstellung vergleichsweise aufwändig ist, da es eine zusätzliche Sulfidierung erfordert und es bei sehr hohen Temperaturen (400°F bis 1000°F) wärmebehandelt werden muss. Zudem wird ein optionaler Gehalt an Chrom und Nickel empfohlen.

Nach alldem ist es bisher nicht gelungen, einen chrom- und nickelfreien Katalysator zu finden, der sich für die Hydrierung von Hydroformylierungsgemischen im großtechnischen Maßstab eignet.

Im Lichte dieses Standes der Technik besteht die Aufgabe der Erfindung besteht darin, einen Katalysator zu entwickeln, der weder Chrom noch Nickel enthält. Andere als karzinogen bekannte Stoffe sollen ebenfalls nicht enthalten sein. Zudem soll er die wirtschaftliche Hydrierung von aus industriellen Oxo-Prozesses stammenden Aldehydgemischen im großtechnischen Maßstab ermöglichen. Hierzu sollte der Katalysator nicht auf kostspielige Edelmetalle wie Ru, Pd oder Pt angewiesen sein. Der Kupferanteil des Katalysators soll möglichst gering sein, um Rohstoffkosten zu senken. Im Interesse der Produktionskosten des Katalysators ist außerdem, dass seine Herstellung bei niedrigen Temperaturen erfolgen kann und der Katalysator nicht sulfidiert werden muss.

Gelöst wurde diese Aufgabe dadurch, dass das Chrom und Nickel bei der Herstellung eines klassischen Cu/Ni/Cr-Systems weggelassen werden, sodass ein Katalysator erhalten wird, auf dessen Trägermaterial lediglich Kupfer als hydrieraktive Komponente vorkommt, nicht aber Chrom und Nickel. Überraschend ist dabei, dass trotz Verzicht auf zwei von drei hydrieraktiven Metallen überhaupt noch ein für den vorgesehenen Zweck funktionsfähiger Katalysator entsteht. Dies setzt allerdings zwingend voraus, dass als Trägermaterial Siliciumdioxid eingesetzt wird und dass der Gehalt an Cu und SiO₂ im aktiven Katalysator in sehr engen Grenzen genau eingestellt wird. Vermutlich ist SiO₂ als Träger deswegen besonders geeignet, das es sehr wenige Brönstedt-saure bzw. Brönstedtbasische Zentren enthält, welche die Nebenproduktbildung beschleunigen.

Gegenstand der Erfindung ist mithin ein Verfahren zur Herstellung von Alkoholen durch Hydrierung von Aldehyden, bei welchem mindestens ein Aldehyd sowie mindestens eine Begleitkomponente enthaltendes Einsatzgemisch in Gegenwart von Wasserstoff mit einem heterogenen Katalysator in Kontakt gebracht wird, wodurch ein Produktgemisch erhalten wird, welches zumindest den dem hydrierten Aldehyd entsprechenden Alkohol sowie zumindest ein Nebenprodukt enthält, wobei der Katalysator ein Trägermaterial sowie darauf aufgebracht Kupfer umfasst, wobei es sich bei dem Trägermaterial um Siliciumdioxid handelt und wobei der Katalysator in aktivierter Form die folgende, sich zu 100 Gew.-% ergänzende Zusammensetzung aufweist:

| | |
|---|---|
| Siliciumdioxid: | von 86 Gew.-% bis 90 Gew.-%; |
| Kupfer: | von 10 Gew.-% bis 14 Gew.-%; |
| Nickel: | von 0 Gew.-ppm bis 50 Gew.-ppm; |
| Chrom: | von 0 Gew.-ppm bis 50 Gew.-ppm; |
| Aluminiumoxid: | von 0 Gew.-ppm bis 1000 Gew.-ppm; |
| Sonstige Stoffe: | von 0 Gew.-ppm bis 1 Gew.-%. |

Als "sonstige Stoffe" sind in diesem Zusammenhang stets chemische Elemente oder deren Verbindungen zu verstehen, die im Katalysator zwar enthalten sind aber explizit in der Aufzählung genannt sind. Im Fall der obigen Zusammensetzung ist "Sonstige Stoffe" also eine Sammelbezeichnung für alle Komponenten außer Si0₂, Cu, Ni, Cr, Al₂O₃.

Beispiele für "sonstige Stoffe" können Carbonate, Hydroxide oder schlicht eingelagertes Wasser sein. In Spuren nachweisbare Edelmetalle wie Pt, Ru oder Pd fallen ebenfalls unter den Begriff "sonstige Stoffe". Aus Kostengründen wird gemäß der Erfindung auf die absichtliche Zugabe von Edelmetallen bei der Herstellung des Katalysators verzichtet. Es ist aber nicht auszuschließen, dass sich mit den heutigen analytischen Möglichkeiten in dem hergestellten Katalysator Spuren von Pt, Ru oder Pd nachweisen lassen. Diese wären dann ebenso wie Rückstände von Nickel und Chrom aber als unbeabsichtigte Verunreinigung (durch Werkstoffe der Apparaturen oder Schmuck des Personals) einzustufen. Idealerweise ist der Gehalt "sonstiger Stoffe" Null.

Als für die Hydrieraufgabe besonders geeignet erwiesen hat sich ein Katalysator, welcher in aktivierter Form die folgende, sich zu 100 Gew.-% ergänzende Zusammensetzung aufweist:

| | |
|---|---|
| Siliciumdioxid: | von 87 Gew.-% bis 89 Gew.-%; |
| Kupfer: | von 11 Gew.-% bis 13 Gew.-%; |
| Nickel: | von 0 Gew.-ppm bis 50 Gew.-ppm; |
| Chrom: | von 0 Gew.-ppm bis 50 Gew.-ppm; |
| Hydroxide: | von 0 Gew.-ppm bis 100 Gew.-ppm; |
| Ruthenium: | von 0 Gew.-ppm bis 50 Gew.-ppm; |
| Palladium: | von 0 Gew.-ppm bis 50 Gew.-ppm; |
| Platin: | von 0 Gew.-ppm bis 50 Gew.-ppm; |
| Aluminiumoxid: | von 0 Gew.-ppm bis 100 Gew.-ppm; |
| Wasser: | von 0 Gew.-ppm bis 100 Gew.-ppm; |
| Carbonate: | von 0 Gew.-ppm bis 100 Gew.-ppm; |
| Sonstige Stoffe: | von 0 Gew.-ppm bis 0.5 Gew.-%. |

Als Trägermaterial wird erfindungsgemäß Siliciumdioxid eingesetzt. Wahlweise kann pyrogenes Si0₂ bzw. Fällungskieselsäure als Trägermaterial verwendet werden. Unabhängig von der SiO₂-Synthese wird der Katalysator immer als Feststoff mit mehr oder minder kristallinem Anteil im Trägermaterial vorliegen. Es handelt sich somit um einen heterogenen Katalysator in der Hydrierung, da er nicht in dem Reaktionsgemisch gelöst wird, sondern in einer anderen Phase vorliegt.

Vorzugweise wird ein Trägermaterial eingesetzt, dessen spezifisches Porenvolumen zwischen 0.8 cm³/g bis 1.2 cm³/g beträgt, bestimmt nach der Cyclohexan-Immersionsmethode, und dessen spezifische Oberfläche (BET-Oberfläche) zwischen 130 m²/g bis 170 m²/g beträgt, bestimmt nach der ISO-Methode 9277. Ein solches Material ist als Aerolyst® 3041 von Evonik Ressource Efficiency GmbH erhältlich. Es basiert auf pyrogenem Siliciumdioxid.

Im Interesse einer hohen Prozessintensität erfolgt die Hydrierung bei einem Druck zwischen 15^{∗}10⁵ Pa und 25^{∗}10⁵ Pa und bei einer Temperatur zwischen 140 °C und 180 °C. Druck und Temperatur sind dabei so zu wählen, dass Einsatzgemisch und Produktgemisch in einer flüssigen Phase vorliegen.

Vorzugsweise wird der Wasserstoff überstöchiometrisch angeboten, um eine möglichst vollständige Hydrierung zu gewährleisten. Die Konzentration des Wasserstoffes ist aber dabei so einzustellen, dass zumindest ein Teil des Wasserstoffs in der flüssigen Phase gelöst vorliegt. Ein Teil des Wasserstoffs kann aber auch in der Gasphase vorliegen und so in dem Reaktionsgemisch Blasen bilden. Die Reaktion erfolgt dann im so genannten "Trickle-Bed"-Betrieb.

Das erfindungsgemäße Katalysatorsystem wurde dafür entwickelt, Einsatzgemische zu hydrieren, welche aus einer Hydroformylierung stammen und als solche mehrere Aldehyde mit derselben Anzahl n an Kohlenstoffatomen sowie entsprechende Alkohole und Hochsieder enthalten, wobei es sich bei n um eine natürliche Zahl zwischen drei und achtzehn handelt.

Besonders optimiert wurde es für die Hydrierung von C₉-Aldehydgemische mit der folgenden sich zu 100 Gew.-% ergänzenden Spezifikation:

| | |
|---|---|
| Gesamtanteil der Aldehyde mit neun Kohlenstoffatomen: | 25 Gew.-% bis 75 Gew.-%; |
| Gesamtanteil der Alkohole mit neun Kohlenstoffatomen: | 10 Gew.-% bis 55 Gew.-%; |
| Gesamtanteil an Acetalen: | 0.5 Gew.-% bis 5.5 Gew.-%; |
| Gesamtanteil weitere Kohlenwasserstoffe: | 0 Gew.-% bis 40 Gew.-%; |
| Wasser: | 0 Gew.-% bis 3 Gew.-%. |

Ein solches Einsatzgemisch entsteht typischerweise bei der Cobalt-katalysierten Hydroformylierung von C₈-Olefinen.

Ebenso geeignet ist es für die Hydrierung von C₉-Aldehydgemische mit der folgenden sich zu 100 Gew.-% ergänzenden Spezifikation:

| | |
|---|---|
| Gesamtanteil der Aldehyde mit neun Kohlenstoffatomen: | 15 Gew.-% bis 65 Gew.-%; |
| Gesamtanteil der Alkohole mit neun Kohlenstoffatomen: | 20 Gew.-% bis 65 Gew.-%; |
| Gesamtanteil an Acetalen: | 0.5 Gew.-% bis 5.5 Gew.-%; |
| Gesamtanteil weitere Kohlenwasserstoffe: | 0 Gew.-% bis 40 Gew.-%; |
| Wasser: | 0 Gew.-% bis 1 Gew.-%. |

Ein solches Einsatzgemisch entsteht typischerweise bei der Rhodium-katalysierten Hydroformylierung von C₈-Olefinen.

Die chemischen und physikalischen Eigenschaften eines Katalysators und damit seine Eignung für die Hydrieraufgabe wird maßgeblich durch seine Herstellung bestimmt.

Mithin ist die Herstellung des Katalysators ein wesentlicher Aspekt der Erfindung. Diese geschieht gemäß den Ansprüchen im Wesentlichen nach den folgenden Schritten:
a) Bereitstellen von einem Trägermaterial, welches mindestens 99 Gew.-% Siliciumdioxid enthält;
b) Bereitstellen von Kupfer(II)hydroxid-carbonat, Ammoniumhydrogencarbonat und/oder Ammoniumcarbonat, Ammoniak und Wasser;
c) Herstellen einer Lösung aus Kupfer(II)hydroxid-carbonat Ammoniumhydrogencarbonat und/oder Ammoniumcarbonat, Ammoniak und Wasser, dergestalt, dass die Lösung einen Kupfer-Anteil zwischen 10 Gew.-% und 15 Gew.-% aufweist, wobei der Anteil an Chrom der Lösung zwischen 0 Gew.-ppm und 50 Gew.-ppm liegt, und wobei der Anteil an Nickel der Lösung zwischen 0 Gew.-ppm und 50 Gew.-ppm liegt;
d) Imprägnieren des Trägermaterials mit der Lösung;
e) Trocknen des imprägnierten Trägermaterials bei Temperaturen zwischen 50 °C und 150 °C;
f) Kalzinieren des getrockneten, imprägnierten Trägermaterials bei Temperaturen zwischen 300 °C und 600 °C unter Erhalt eines Präkursors;
g) Aktivierung des Präkursors durch Reduktion mit Wasserstoff unter Erhalt des aktiven Katalysators.

Genauer gesagt, beziehen sich die Schritte a) bis f) auf die Herstellung des Präkursors und Schritt g) auf die Herstellung des eigentlichen Katalysators aus dem Präkursor.

Diese Unterscheidung ist beachtlich, da die Aktivierung oft an einem anderen Ort erfolgt als die Herstellung des Präkursors. Meist erfolgt die Aktivierung in-situ, also an dem Ort, an dem später hydriert wird, genauer gesagt im Reaktor. In einem solchen Fall wird der nicht katalytisch aktive Präkursor in den Hydrierreaktor eingebaut, sodann zwecks Reduktion mit Wasserstoff beaufschlagt und so aktiviert. Das hat den Vorteil, dass durch Kontakt mit Luftsauerstoff nicht wieder Kupferoxide gebildet werden.

Alternativ wird der Katalysator ex-situ aktiviert, also außerhalb des Hydrierreaktors reduziert und in aktiver Form angeliefert und eingebaut. Dies muss dann aber unter Schutzatmosphäre erfolgen, was entsprechend aufwändig ist.

Kupfer wird in dem Katalysator als hydrieraktives Metall benötigt. Im Interesse der Katalysatorkosten ist der Kupfergehalt jedoch auf das notwendige Minimum zu reduzieren. Aus diesem Grund empfiehlt es sich bei der Herstellung der Lösung gemäß Schritt c) die Lösung so anzusetzen, dass der Kupfer-Gehalt zwischen 10.5 Gew.-% und 11.5 Gew.-% liegt. Optimal hat sich eine Lösung mit Kupfergehalt von 11 Gew-% erwiesen.

Die Imprägnierung des Trägermaterials mit der Lösung kann auf unterschiedliche Weisen erfolgen. Die Lösung soll in die Poren eindringen und diese weitestgehend füllen. Folgendes Vorgehen bei der Imprägnierung hat sich bewährt: In eine rotierende Trommel wird der Träger eingebracht. Die Lösung wird auf den Träger gesprüht, füllt die Poren. Während der Sprühphase kann durch die rotierende Schüttung des imprägnierten Trägers warme Luft geleitet werden. Bei der warmen Luft handelt es sich in der Regel um Luft mit einer Temperatur, die im Bereich der Raumtemperatur liegt.

Das Imprägnieren gemäß Schritt d) und zumindest ein Teil des Trocknens des imprägnierten Trägermaterials gemäß Schritt e) erfolgt demnach erfindungsgemäß in einer Trommel, dergestalt, dass das Trägermaterial zum Imprägnieren in die Trommel eingebracht wird, dass die Trommel rotiert wird, dass die Lösung in die Trommel eingespritzt wird, und dass während des Trocknens ein Luftstrom mit einer Temperatur zwischen 50 °C und 100 °C , vorzugsweise mit einer Temperatur von ungefähr 80 °C, durch die Trommel geleitet wird. Schritte d) und e) werden somit mit wenig manuellen Aufwand in einer Apparatur (der Trommel) vorgenommen. Dies senkt die Produktionskosten.

In einer weiteren Ausführungsform der vorliegenden Erfindung ist es ebenfalls möglich das imprägnierte Trägermaterial vor Abschluss der Trocknung aus der Trommel zu entnehmen und in einem Trockner bei einem Luftstrom im Temperaturbereich von 100 °C bis 150 °C fertig zu trocknen. Das Umladen aus der Trommel in den Trockner ist zwar ein zusätzlicher Produktionsschritt, kann aber zu einem geringen Wassergehalt führen.

Die Handhabung des Katalysators bei seiner Herstellung und beim Einbau in den Reaktor wird wesentlich erleichtert, wenn es sich bei dem bereitgestellten Trägermaterial um zylindrische Extrudate mit einem Durchmesser zwischen 1 mm und 2 mm handelt. Der Katalysator kann dann wie ein Schüttgut gehandhabt werden. In der Flüssigphasenhydrierung sind die zylindrischen Extrudate strömungsdynamisch günstig. Aerolyst® 3014 von Evonik ist in dieser bevorzugten Darreichungsform erhältlich. Als alterative Katalysatorform können auch Kugeln im Durchmesserbereich zwischen 1 mm und 2 mm eingesetzt werden.

Vor der Aktivierung weist der Präkursor vorzugweise die folgende, sich zu 100 Gew.-% ergänzende Zusammensetzung auf:

| | |
|---|---|
| Siliciumdioxid: | von 84 Gew.-% bis 86 Gew.-%; |
| Kupferoxid: | von 14 Gew.-% bis 16 Gew.-%; |
| Nickel: | von 0 Gew.-ppm bis 50 Gew.-ppm; |
| Chrom: | von 0 Gew.-ppm bis 50 Gew.-ppm; |
| Aluminiumoxid: | von 0 Gew.-ppm bis 1000 Gew.-ppm; |
| Sonstige Stoffe: | von 0 Gew.-ppm bis 1 Gew.-%. |

Beachtlich ist, dass das Kupfer in dieser Vorstufe noch oxidisch vorliegt. Aufgrund des gebundenen Sauerstoffs unterscheidet sich das Gesamtgewicht des Präkursors von dem aktiven Katalysator, weswegen auch die relativen Mengenangaben abweichen.

Bei der Aktivierung wird das CuO mit H₂ zu Cu reduziert. Das dabei entstehende Wasser wird abgezogen. Dank des nun metallischen Kupferkontakts wird der Katalysator nun hydrieraktiv. Nach Abschluss der Aktivierung ist der Sauerstoff entfernt, sodass der aktive Katalysator die erfindungsgemäße Zusammensetzung aufweist. Vorzugsweise wird der Katalysator in-situ mit flüssigem Wasserstoff reduziert. Der Katalysator kann aber auch gut in der Gasphase aktiviert werden.

Die Verwendung der so hergestellten Katalysatoren in Verfahren zur Herstellung von Alkoholen durch Hydrierung von Aldehyden gemäß der vorliegenden Erfindung ist ebenfalls Gegenstand der Erfindung.

Die beschriebene Herstellung des Präkursors, dessen Aktivierung und der Einsatz des aktiven Katalysators in der Hydrierung von Aldehyden lösen gemeinschaftlich die gesetzte Aufgabe.

Somit ergibt sich ein kombiniertes Verfahren zur Herstellung eines Präkursors, Aktvierung des Präkursors zu einem aktiven Katalysator und Verwendung des Katalysators zur Hydrierung von Aldehyden. Das Verfahren zur Hydrierung von Aldehyden umfasst demgemäß die folgenden Schritte:
a) Bereitstellen von einem Trägermaterial, welches mindestens 99 Gew.-% Siliciumdioxid enthält;
b) Bereitstellen von Kupfer(II)hydroxid-carbonat, Ammoniumhydrogencarbonat und/oder Ammoniumcarbonat, Ammoniak und Wasser;
c) Herstellen einer Lösung enthaltend Kupfer(II)hydroxid-carbonat, Ammoniumhydrogencarbonat und/oder Ammoniumcarbonat, Ammoniak und Wasser, dergestalt, dass die Lösung einen Kupfer- Anteil zwischen 10 Gew.-% und 15 Gew.-% aufweist, wobei der Anteil an Chrom der Lösung zwischen 0 Gew.-ppm und 50 Gew.-ppm liegt, und wobei der Anteil an Nickel der Lösung zwischen 0 Gew.-ppm und 50 Gew.-ppm liegt;
d) Imprägnieren des Trägermaterials mit der Lösung;
e) Trocknen des imprägnierten Trägermaterials bei Temperaturen zwischen 50 °C und 150 °C;
f) Kalzinieren des getrockneten, imprägnierten Trägermaterials bei Temperaturen zwischen 300 °C und 600 °C unter Erhalt eines Präkursors, welcher insbesondere folgende, sich zu 100 Gew.-% ergänzende Zusammensetzung aufweist:

| | |
|---|---|
| Siliciumdioxid: | von 84 Gew.-% bis 86 Gew.-%; |
| Kupferoxid: | von 14 Gew.-% bis 16 Gew.-%; |
| Nickel: | von 0 Gew.-ppm bis 50 Gew.-ppm; |
| Chrom: | von 0 Gew.-ppm bis 50 Gew.-ppm; |
| Aluminiumoxid: | von 0 Gew.-ppm bis 1000 Gew.-ppm; |
| Sonstige Stoffe: | von 0 Gew.-ppm bis 1 Gew.-%. |

g) Verbringen des Präkursors in einen Reaktor;
h) Aktivierung des Präkursors im Reaktor durch Reduktion mit Wasserstoff unter Erhalt des aktiven Katalysators, welcher insbesondere die folgende, sich zu 100 Gew.-% ergänzende Zusammensetzung aufweist:

| | |
|---|---|
| Siliciumdioxid: | von 86 Gew.-% bis 90 Gew.-%; |
| Kupfer: | von 10 Gew.-% bis 14 Gew.-%; |
| Nickel: | von 0 Gew.-ppm bis 50 Gew.-ppm; |
| Chrom: | von 0 Gew.-ppm bis 50 Gew.-ppm; |
| Aluminiumoxid: | von 0 Gew.-ppm bis 1000 Gew.-ppm; |
| Sonstige Stoffe: | von 0 Gew.-ppm bis 1 Gew.-%. |

i) Beaufschlagen des aktiven Katalysators im Reaktor mit einem Einsatzgemisch enthaltend mindestens ein Aldehyd sowie mindestens eine Begleitkomponente in Gegenwart von Wasserstoff, wodurch insbesondere ein Produktgemisch erhalten wird, welches zumindest den dem hydrierten Aldehyd entsprechenden Alkohol sowie zumindest ein Nebenprodukt enthält, wobei das Imprägnieren und zumindest ein Teil des Trocknens in einer Trommel erfolgt, dergestalt, dass das Trägermaterial zum Imprägnieren in die Trommel eingebracht wird, dass die Trommel rotiert wird, dass die Lösung in die Trommel eingespritzt wird, und dass während des Trocknens ein Luftstrom mit einer Temperatur zwischen 50°C und 100°C durch die Trommel geleitet wird.

Die Erfindung soll nun anhand von Beispielen näher erläutert werden. Hierfür zeigen:
- Figur 1:: Anlagenfließbild der verwendeten Hochdruck Batch-Hydrierapparatur
- Figur 2:: Umsatz- und Selektivitätsverlauf bei der Batch-Hydrierung einer C9-Aldehyd-Mischung mit einem chrom- und nickelhaltigen Katalysator

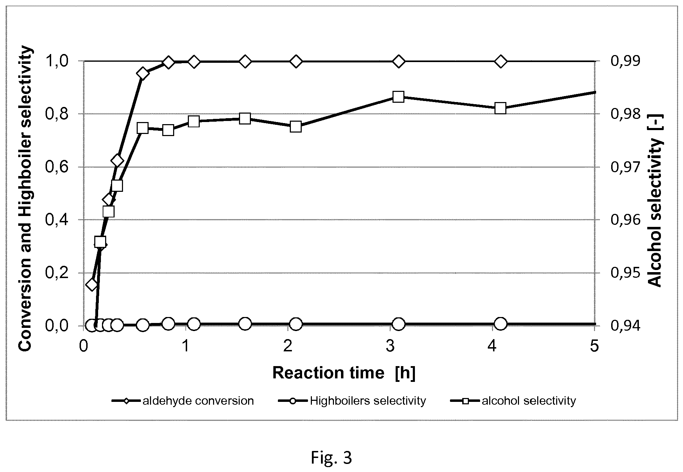

- Figur 3:: Umsatz- und Selektivitätsverlauf bei der Batch-Hydrierung einer C9-Aldehyd-Mischung mit einem chrom- und nickelfreien Katalysator ("Katalyator A")
- Figur 4:: Umsatz- und Selektivitätsverlauf bei der kontinuierlichen Hydrierung einer C9-Aldehyd-Mischung mit einem chrom- und nickelhaltigen Katalysator A sowie einem chrom- und nickelfreien Katalysator C.

### Beispiel 0: Herstellung eines Präkursors eines chromfreien und nickelfreien Cu/SiO2-Katalysators

Ein SiO₂-Träger wird bei Umgebungstemperatur mit einer wässrigen Kupfertetrammincarbonatlösung (erfindungsgemäß hergestellt aus Kupfer(II)hydroxid-carbonat, Ammoniumhydrogencarbonat und/oder Ammoniumcarbonat, Ammoniak und Wasser) imprägniert. Sodann erfolgt eine Trocknung im Luftstrom bei moderaten Temperaturen. Abschließend wird in Luft bei 450 °C kalziniert.

Als Trägermaterial wird Aerolyst® 3041 von Evonik eingesetzt. Dessen typische Eigenschaften sind ein SiO₂-Gehalt größer 99 %, zylinderische Extrudate mit Durchmessern um die 1.7 mm, Porenvolumina von ungefähr1 cm³/g und BET Oberflächen von ungefähr 150 m²/g.

Aus Kupferhydroxidcarbonat, konzentrierte Ammoniaklösung, Ammoniumhydrogencarbonat und/oder Ammoniumcarbonat und Wasser wird eine Kupfertetrammincarbonatlösung hergestellt, die etwa 13 Gew.-% Kupfer enthielt. Die Lösung wurde anschließend nochmal mit Wasser auf etwa 11 Gew.-% Kupfer verdünnt.

Zwecks Imprägnierung wurde die verdünnte Kupfertetrammincarbonatlösung anschließend auf das Trägermaterial gesprüht. Bei der Tränkung rotiert der Träger in einer Trommel. Während des Sprühvorgangs wurde eine gewisse Menge an Luft durch das Festkörperbett geleitet. Die aufgebrachte Menge an Sprühlösung ist so bemessen, dass nach der Imprägnierung nahezu alle Poren mit Flüssigkeit gefüllt sind und die Kupfersalzlösung im ganzen Korn verteilt ist.

Die Trocknung erfolgte in derselben Trommel. Dazu wurde der Luftstrom, der durch die Trommel und Festkörperschüttung geleitet wird, auf Temperaturen von etwa 75 °C aufgeheizt. In diesem speziellen Fall wurde der Katalysator vor der Kalzination noch bei 120 °C im Luftstrom in einem weiteren Reaktor nachgetrocknet.

Die Kalzination, bei dem das Kupfersalz im Wesentlichen zum Kupferoxid umgesetzt wird, erfolgte in einem kontinuierlich mit Luft durchströmten Ofen, die GHSV (gas hourly space velocity) betrug 2000 h⁻¹, der Katalysator wurde bei 450 °C für 10 h in Luft kalziniert

Prinzipiell kann der Katalysator nach allen Imprägnierverfahren hergestellt werden, bei denen die Poren des Trägers mit der Lösung gefüllt werden, dazu gehören beispielsweise auch Vakuumimprägnierung oder das Überschütten des Trägers mit überschüssiger Lösung.

Die Trocknung kann auch in anderen üblichen industriellen Aggregaten erfolgen (Schachttrockner, Kammerofen, Bandtrockner, Trommeltrockner, Vakuumtrockner), typische Trocknungstemperaturen liegen zwischen 50 °C und 150 °C

Auch bei der Kalzination können unterschiedliche Aggregate verwendet werden: Schachtöfen, Kammeröfen, Drehrohröfen usw. Die Temperatur von 450 °C ist sicherlich auch nicht extrem ausschlaggebend, auch hier sind etwas niedrigere Temperaturen als auch höhere Temperaturen denkbar.

Der auf diese Weise hergestellte Präkursor enthielt 15 Gew.-% CuO und etwa 85 Gew.-% SiO₂. Zwecks Aktivierung zum Katalysator wird der Präkursor mit einem gasförmigen Wasserstoffstrom bei 10^{∗}10⁵ Pa mit 60 NI/h (STP) beaufschlagt. Dabei wurde die Temperatur stündlich in 30 K-Schritten von 60 °C auf 180 °C erhöht und für 12 h konstant gehalten.

### Beispiel 1 (nicht erfindungsgemäß): Hydrierung von C₉-Aldehyden aus einer Co-katalysierten Hydroformylierung bei 180 °C mit einem chrom- und nickelhaltigen Katalysator

Als Katalysator A wird ein chromhaltiger Katalysator eingesetzt, wie er auch für die in DE19842370A1 beschrieben Versuche verwendet wurde.

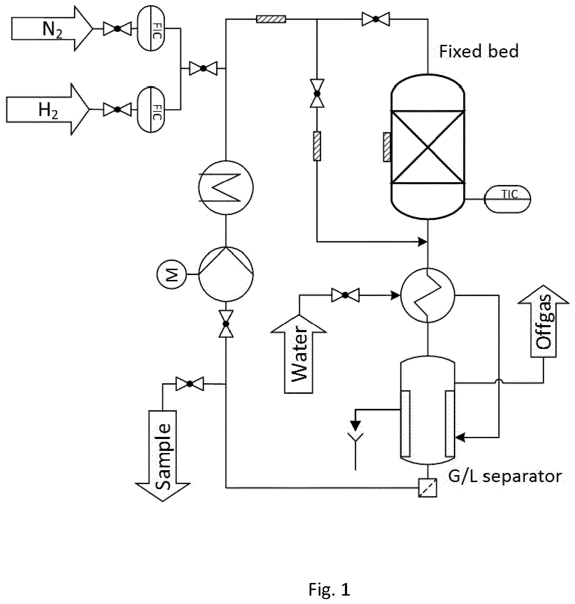

Für die Durchführung der Batch-Hydrierversuche wurde ein 1 L, durch externen Kreislauf gerührter, Hochdruck-Edelstahlreaktor verwendet. Der Reaktor verfügt über einen Einsatz zur Katalysatorbefüllung mit einem Durchmesser von 4 cm und einer Länge von 30 cm. Ein vereinfachtes Fließbild der Anlage ist in Figur 1 dargestellt.

Die Beheizung des Reaktors und der Leitungen erfolgte über gewickelte Heizbänder, die Kontrolle und Regelung der Temperatur über PT100-Thermoelemente. Die Flüssigphase wurde über eine Gather DS2X30 Zahnradpumpe mit Kreislaufgeschwindigkeiten von 45 L/h umgewälzt. Die Temperierung der Flüssigphase erfolgte mit Hilfe eines LAUDA LTH350s Thermostaten. Als Wärmeträger kam MARLOTHERM SH zum Einsatz. Für die Trennung der Phasen wurde ein Edelstahlbehälter (Innenvolumen 2 L) installiert und mit Wasser kontinuierlich gekühlt. Der Phasentrenner diente gleichzeitig als Vorlage für das flüssige Edukt. Die Wasserstoff- und Stickstoffzufuhr wurden über Bronkhorst F231M bzw. F231C Massendurchflussmesser geregelt. Vor der Durchführung von Batchexperimenten wurden 90mL Katalysator A (trocken) in den Katalysatorkorb eingefüllt und im Reaktor eingebaut. Die Reduktion der frischen Hydrierkatalysatoren erfolgte im Wasserstoffstrom bei 10^{∗}10⁵ Pa mit 60 NI/h. Dabei wurde die Temperatur stündlich in 30 K-Schritten von 60 °C auf 180 °C erhöht und für 12 h konstant gehalten. Der Phasentrenner wurde mit 1 L Edukt befüllt und die Flüssigphase im Kreislauf über den Reaktorbypass aufgeheizt. Nach Erreichen der gewünschten Reaktionstemperatur wurde eine Bypassprobe genommen und die Reaktion durch Öffnen des Reaktorkugelhahns gestartet. Während der Reaktion wurden zu definierten Zeiten Proben genommen und via offline-GC (7890B GC; FA Agilent Technologie) analysiert.

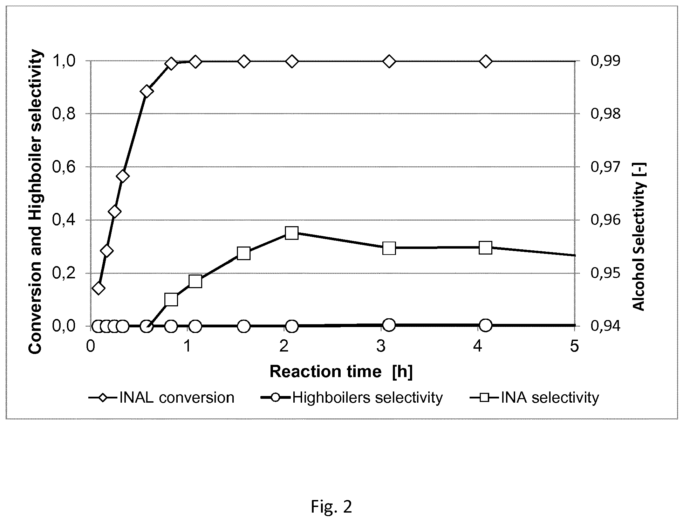

Die in diesem Versuch erzielten Umsätze und Selektivitäten sind in Figur 2 graphisch dargestellt.

### Beispiel 2 (erfindungsgemäß): Hydrierung von C₉-Aldehyden aus einer Co-katalysierten HyFo bei 180°C mit einem chrom- und nickelfreien Katalysator

Die Batch-Hydrierung mit dem chrom- und nickelfreien Katalysator aus Beispiel 0 ("Katalysator C") wird wie in Beispiel 1 beschrieben durchgeführt. Dabei wird Katalysator A durch Katalysator C ersetzt. Der Umsatz- und Selektivitätsverlauf zeigt, dass mit Katalysator C eine deutlich höhere Alkohol-Selektivität erzielt werden kann.

### Beispiel 3: Langzeitvergleich eines chrom- und nickelhaltigen mit einem chrom- und nickelfreien Katalysators

Die parallelisierte Evaluierung alternativer Hydrierkatalysatoren erfolgte in einer kontinuierlich betriebenen 16-fach Anlage zur Testung heterogener Katalysatorsysteme. Die Anlage verfügt über einen zentralen Reaktorblock mit 16 separaten Edelstahlreaktoren mit einer Länge von 96 cm und einem Innendurchmesser von 5 mm. Die Beheizung des Reaktorblocks erfolgt elektrisch und erlaubt einen isothermen Betrieb der Reaktoren bei einer Genauigkeit von ±1 °C. Über ein System aus Kapillarrestriktoren erfolgt die Verteilung und Dosierung der flüssigen und gasförmigen Edukte, dabei basiert die Druckhaltung der Reaktoren auf Gegendruckmembranmodulen der Firma Equilibar. Für die Evaluation der Hydrierkatalysatoren wurden, nach Abschätzung der Katalysatoraktivität aus den in den Beispielen 1 und 2 beschriebenen Batch-Versuchen, 0.2 g bis 0.6 g Katalysator (trocken) als Siebfraktion (0.2 mm bis 0.4 mm) eingesetzt und mit Quarzbruch (0.3 mm bis 0.5 mm) verdünnt. Die Verdünnung der eingesetzten Katalysatoren erfolgte immer so, dass eine konstante Länge des katalytisch aktiven Betts von 10 cm erzeugt wurde. Die Flüssigphasenhydrierung wird im Trickle-Bed-Betrieb durchgeführt; dabei muss Wasserstoff gasförmig zugeführt werden. Der Wasserstoff liegt dann teilweise in der flüssigen Phase gelöst und teilweise als Blasen in der Gasphase vor. Zudem wird mit Abgas gefahren, um die Akkumulation gasförmiger Nebenprodukte zu vermeiden. Die Testung erfolgte mithin bei einer Temperatur von 180 °C und einem Wasserstoffdruck von 25^{∗}10⁵ Pa bei einer LHSV (liquid hourly space velocity) von 6 h⁻¹ und einer GHSV (gas hourly space velocity des gasförmigen Wasserstoffes) von 2000 h⁻¹. Alle Messungen erfolgten als Doppelbestimmung. Die Analyse der Produktproben erfolgte per offline-GC (7890B GC; FA Agilent Technologie), analog zu den Hydrierversuchen gemäß Beispiel 1 und Beispiel 2.

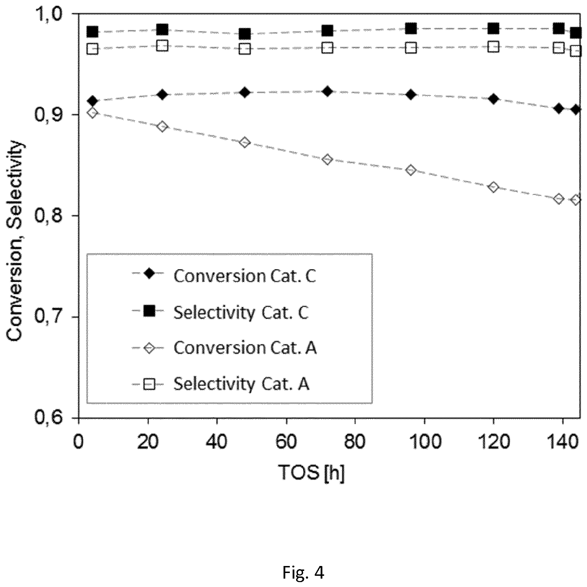

Wie der in Figur 4 dargestellte Umsatz- und Selektivitätsverlauf dieses Vergleichs zeigt, kann mit dem erfindungsgemäßen chrom- und nickelfreien Katalysator C eine gleichbleibend hohe Alkohol-Selektivität erzielt werden, die im gesamten Versuchszeitraum über derjenigen des chrom- und nickelhaltigen Vergleichskatalysators A liegt. Zugleich zeichnet sich Katalysator C gegenüber Katalysator A durch eine deutlich geringere Abnahme des Umsatzgrads aus.

Der Vergleich der Beispiele zeigt, dass mit dem erfindungsgemäßen Cr- und Ni-freien Cu-Katalysator sich aus technischer Hydroformylierung erhaltene C₅, C₉, C₁₃ und C₁₇-Aldehydgemische hydrieren lassen und dabei Produktgemische erhalten werden, die einen hohen Anteil an den korrespondierenden Aldehyden enthalten, während der Anteil der unerwünschten Nebenprodukte gering ist.

Über einen längeren Zeitraum gesehen sind die Aldehyd-Ausbeuten der chrom- und nickelfreien Versuche nicht wesentlich schlechter als die Vergleichsversuche mit klassischen Ni/Cu/Cr-Systemen.

Bei Herstellung des chrom- und nickelfreien Katalysators kann der Umgang mit kanzerogenen Substanzen vermieden werden. Der Einsatz von Edelmetallen ist nicht notwendig. Zusätzliche Arbeitsschritte bei der Herstellung des Katalysators, wie zum Beispiel eine Sulfidierung, waren nicht erforderlich. Bei der Herstellung waren keine hohen Temperaturen notwendig. Die Hydrierergebnisse sind vollauf zufriedenstellend. Der Katalysator erscheint industriell wirtschaftlich einsetzbar.

## Patentansprüche

1. Verfahren zur Herstellung von Alkoholen durch Hydrierung von Aldehyden, bei welchem mindestens ein Aldehyd sowie mindestens eine Begleitkomponente enthaltendes Einsatzgemisch in Gegenwart von Wasserstoff mit einem heterogenen Katalysator in Kontakt gebracht wird, wodurch ein Produktgemisch erhalten wird, welches zumindest den dem hydrierten Aldehyd entsprechenden Alkohol sowie zumindest ein Nebenprodukt enthält, wobei der Katalysator ein Trägermaterial sowie darauf aufgebracht Kupfer umfasst,
**dadurch gekennzeichnet,**
**dass** es sich bei dem Trägermaterial um Siliciumdioxid handelt;
und **dass** der Katalysator in aktivierter Form die folgende, sich zu 100 Gew.-% ergänzende Zusammensetzung aufweist:
| | |
|---|---|
| Siliciumdioxid: | von 86 Gew.-% bis 90 Gew.-%; |
| Kupfer: | von 10 Gew.-% bis 14 Gew.-%; |
| Nickel: | von 0 Gew.-ppm bis 50 Gew.-ppm; |
| Chrom: | von 0 Gew.-ppm bis 50 Gew.-ppm; |
| Aluminiumoxid: | von 0 Gew.-ppm bis 1000 Gew.-ppm; |
| Sonstige Stoffe: | von 0 Gew.-ppm bis 1 Gew.-%. |

2. Verfahren nach Anspruch 1, **dadurch gekennzeichnet, dass** der Katalysator in aktivierter Form die folgende, sich zu 100 Gew.-% ergänzende Zusammensetzung aufweist:
| | |
|---|---|
| Siliciumdioxid: | von 87 Gew.-% bis 89 Gew.-%; |
| Kupfer: | von 11 Gew.-% bis 13 Gew.-%; |
| Nickel: | von 0 Gew.-ppm bis 50 Gew.-ppm; |
| Chrom: | von 0 Gew.-ppm bis 50 Gew.-ppm; |
| Ruthenium: | von 0 Gew.-ppm bis 50 Gew.-ppm; |
| Palladium: | von 0 Gew.-ppm bis 50 Gew.-ppm; |
| Platin: | von 0 Gew.-ppm bis 50 Gew.-ppm; |
| Aluminiumoxid: | von 0 Gew.-ppm bis 100 Gew.-ppm; |
| Wasser: | von 0 Gew.-ppm bis 100 Gew.-ppm; |
| Carbonate: | von 0 Gew.-ppm bis 100 Gew.-ppm; |
| Hydroxide: | von 0 Gew.-ppm bis 100 Gew.-ppm; |
| Sonstige Stoffe: | von 0 Gew.-ppm bis 0.5 Gew.-%. |

3. Verfahren nach Anspruch 1 oder Anspruch 2, **dadurch gekennzeichnet, dass** das spezifische Porenvolumen des Trägermaterials zwischen 0.8 cm³/g bis 1.2 cm³/g beträgt, bestimmt nach der Cyclohexan-Immersionsmethode, und dass die spezifische Oberfläche des Trägermaterials (BET-Oberfläche) zwischen 130 m²/g bis 170 m²/g beträgt, bestimmt nach der ISO-Methode 9277.

4. Verfahren nach Anspruch 1 oder nach einem der Ansprüche 2 bis 3, **dadurch gekennzeichnet, dass** es bei einem Druck zwischen 15^{∗}10⁵ Pa und 25^{∗}10⁵ Pa und bei einer Temperatur zwischen 140 °C und 180 °C durchgeführt wird, wobei Druck und Temperatur so gewählt sind, dass Einsatzgemisch und Produktgemisch in einer flüssigen Phase vorliegen.

5. Verfahren nach Anspruch 4, **dadurch gekennzeichnet, dass** der Wasserstoff überstöchiometrisch gegenwärtig ist, wobei die Konzentration des Wasserstoffes so gewählt ist, dass zumindest ein Teil des Wasserstoffs in der flüssigen Phase gelöst vorliegt.

6. Verfahren nach Anspruch 1 oder nach einem der Ansprüche 2 bis 5, **dadurch gekennzeichnet, dass** das Einsatzgemisch aus einer Hydroformylierung stammt und als solches mehrere Aldehyde mit derselben Anzahl n an Kohlenstoffatomen sowie entsprechende Alkohole und Hochsieder enthält, wobei es sich bei n um eine natürliche Zahl zwischen drei und achtzehn handelt.

7. Verfahren nach Anspruch 6, **dadurch gekennzeichnet, dass** das Einsatzgemisch die folgende, sich zu 100 Gew.-% ergänzende Zusammensetzung aufweist:
| | |
|---|---|
| Gesamtanteil der Aldehyde mit neun Kohlenstoffatomen: | 25 Gew.-% bis 75 Gew.-%; |
| Gesamtanteil der Alkohole mit neun Kohlenstoffatomen: | 10 Gew.-% bis 55 Gew.-%; |
| Gesamtanteil an Acetalen: | 0.5 Gew.-% bis 5.5 Gew.-%; |
| Gesamtanteil weitere Kohlenwasserstoffe: | 0 Gew.-% bis 40 Gew.-%; |
| Wasser: | 0 Gew.-% bis 3 Gew.-%. |

8. Verfahren nach Anspruch 6, **dadurch gekennzeichnet, dass** das Einsatzgemisch die folgende, sich zu 100 Gew.-% ergänzende Zusammensetzung aufweist:
| | |
|---|---|
| Gesamtanteil der Aldehyde mit neun Kohlenstoffatomen: | 15 Gew.-% bis 65 Gew.-%; |
| Gesamtanteil der Alkohole mit neun Kohlenstoffatomen: | 20 Gew.-% bis 65 Gew.-%; |
| Gesamtanteil an Acetalen: | 0.5 Gew.-% bis 5.5 Gew.-%; |
| Gesamtanteil weitere Kohlenwasserstoffe: | 0 Gew.-% bis 40 Gew.-%; |
| Wasser: | 0 Gew.-% bis 1 Gew.-%. |

9. Verfahren zur Herstellung eines Katalysators mit den folgenden Schritten:
a) Bereitstellen von einem Trägermaterial, welches mindestens 99 Gew.-% Siliciumdioxid enthält;
b) Bereitstellen von Kupfer(II)hydroxid-carbonat, Ammoniumhydrogencarbonat und/oder Ammoniumcarbonat, Ammoniak und Wasser;
c) Herstellen einer Lösung aus Kupfer(II)hydroxid-carbonat, Ammoniumhydrogencarbonat und/oder Ammoniumcarbonat, Ammoniak und Wasser, dergestalt, dass der Kupfer-Anteil an der Lösung zwischen 10 Gew.-% und 15 Gew.-% liegt, wobei der Anteil an Chrom der Lösung zwischen 0 Gew.-ppm und 50 Gew.-ppm liegt, und wobei der Anteil an Nickel der Lösung zwischen 0 Gew.-ppm und 50 Gew.-ppm liegt;
d) Imprägnieren des Trägermaterials mit der Lösung;
e) Trocknen des imprägnierten Trägermaterials bei Temperaturen zwischen 50 °C und 150 °C;
f) Kalzinieren des getrockneten, imprägnierten Trägermaterials bei Temperaturen zwischen 300 °C und 600 °C unter Erhalt eines Präkursors;
g) Aktivierung des Präkursors durch Reduktion mit Wasserstoff unter Erhalt des aktiven Katalysators,
**dadurch gekennzeichnet, dass** das Imprägnieren und zumindest ein Teil des Trocknens in einer Trommel erfolgt, dergestalt, dass das Trägermaterial zum Imprägnieren in die Trommel eingebracht wird, dass die Trommel rotiert wird, dass die Lösung in die Trommel eingespritzt wird, und dass während des Trocknens ein Luftstrom mit einer Temperatur zwischen 50 °C und 100 °C durch die Trommel geleitet wird.

10. Verfahren nach Anspruch 9, **dadurch gekennzeichnet, dass** der Kupfer-Gehalt der Lösung zwischen 10.5 Gew.-% und 11.5 Gew.-% beträgt.

11. Verfahren nach einem der Ansprüche 9 bis 10, **dadurch gekennzeichnet, dass** es sich bei dem bereitgestellten Trägermaterial um zylindrische Extrudate mit einem Durchmesser zwischen 1 mm und 2 mm handelt.

12. Verfahren nach einem der Ansprüche 9 bis 11, **dadurch gekennzeichnet, dass** der Präkursor die folgende, sich zu 100 Gew.-% ergänzende Zusammensetzung aufweist:
| | |
|---|---|
| Siliciumdioxid: | von 84 Gew.-% bis 86 Gew.-%; |
| Kupferoxid: | von 14 Gew.-% bis 16 Gew.-%; |
| Nickel: | von 0 Gew.-ppm bis 50 Gew.-ppm; |
| Chrom: | von 0 Gew.-ppm bis 50 Gew.-ppm; |
| Aluminiumoxid: | von 0 Gew.-ppm bis 1000 Gew.-ppm; |
| Sonstige Stoffe: | von 0 Gew.-ppm bis 1 Gew.-% |

13. Verwendung der gemäß einem der Ansprüche 9 bis 12 hergestellten Katalysatoren in Verfahren zur Herstellung von Alkoholen durch Hydrierung von Aldehyden gemäß einem oder mehreren der Ansprüche 1 bis 8.

## Claims

1. Process for preparing alcohols by hydrogenation of aldehydes, in which a feed mixture comprising at least one aldehyde and at least one accompanying component is contacted with a heterogeneous catalyst in the presence of hydrogen, giving a product mixture comprising at least the alcohol corresponding to the hydrogenated aldehyde, and at least one by-product, wherein the catalyst comprises a support material and copper applied thereto,
**characterized in that**
the support material is silicon dioxide;
and the catalyst in activated form has the following composition that adds up to 100% by wt.:
| | |
|---|---|
| silicon dioxide: | from 86% by wt. to 90% by wt.; |
| copper: | from 10% by wt. to 14% by wt.; |
| nickel: | from 0 ppm by wt. to 50 ppm by wt.; |
| chromium: | from 0 ppm by wt. to 50 ppm by wt.; |
| aluminium oxide: | from 0 ppm by wt. to 1000 ppm by wt.; |
| other substances: | from 0 ppm by wt. to 1% by wt. |

2. Process according to Claim 1, **characterized in that** the catalyst in activated form has the following composition that adds up to 100% by wt.:
| | |
|---|---|
| silicon dioxide: | from 87% by wt. to 89% by wt.; |
| copper: | from 11% by wt. to 13% by wt.; |
| nickel: | from 0 ppm by wt. to 50 ppm by wt.; |
| chromium: | from 0 ppm by wt. to 50 ppm by wt.; |
| ruthenium: | from 0 ppm by wt. to 50 ppm by wt.; |
| palladium: | from 0 ppm by wt. to 50 ppm by wt.; |
| platinum: | from 0 ppm by wt. to 50 ppm by wt.; |
| aluminium oxide: | from 0 ppm by wt. to 100 ppm by wt.; |
| water: | from 0 ppm by wt. to 100 ppm by wt.; |
| carbonates: | from 0 ppm by wt. to 100 ppm by wt.; |
| hydroxides: | from 0 ppm by wt. to 100 ppm by wt.; |
| other substances: | from 0 ppm by wt. to 0.5% by wt. |

3. Process according to Claim 1 or Claim 2, **characterized in that** the specific pore volume of the support material is between 0.8 cm³/g and 1.2 cm³/g, determined by the cyclohexane immersion method, and **in that** the specific surface area of the support material (BET surface area) is between 130 m²/g and 170 m²/g, determined by ISO method 9277.

4. Process according to Claim 1 or according to either of Claims 2 and 3, **characterized in that** it is conducted at a pressure between 15^{∗}10⁵ Pa and 25^{∗}10⁵ Pa and at a temperature between 140°C and 180°C, the pressure and temperature being chosen such that feed mixture and product mixture are in a liquid phase.

5. Process according to Claim 4, **characterized in that** the hydrogen is present in a superstoichiometric amount, the concentration of the hydrogen being chosen such that at least some of the hydrogen is dissolved in the liquid phase.

6. Process according to Claim 1 or according to any of Claims 2 to 5, **characterized in that** the feed mixture originates from a hydroformylation and as such comprises a plurality of aldehydes with the same number n of carbon atoms, and corresponding alcohols and high boilers, where n is a natural number between three and eighteen.

7. Process according to Claim 6, **characterized in that** the feed mixture has the following composition that adds up to 100% by wt.:
total fraction of the aldehydes having nine carbon atoms: 25% by wt. to 75% by wt.;
total fraction of the alcohols having nine carbon atoms: 10% by wt. to 55% by wt.;
total fraction of acetals: 0.5% by wt. to 5.5% by wt.;
total fraction of further hydrocarbons: 0% by wt. to 40% by wt.;
water: 0% by wt. to 3% by wt.

8. Process according to Claim 6, **characterized in that** the feed mixture has the following composition that adds up to 100% by wt.:
total fraction of the aldehydes having nine carbon atoms: 15% by wt. to 65% by wt.;
total fraction of the alcohols having nine carbon atoms: 20% by wt. to 65% by wt.;
total fraction of acetals: 0.5% by wt. to 5.5% by wt.;
total fraction of further hydrocarbons: 0% by wt. to 40% by wt.;
water: 0% by wt. to 1% by wt.

9. Process for preparing a catalyst, comprising the following steps:
a) providing a support material containing at least 99% by wt. of silicon dioxide;
b) providing copper(II) hydroxide carbonate, ammonium hydrogencarbonate and/or ammonium carbonate, ammonia and water;
c) preparing a solution from copper(II) hydroxide carbonate, ammonium hydrogencarbonate and/or ammonium carbonate, ammonia and water, in such a way that the copper content of the solution is between 10% by wt. and 15% by wt., where the proportion of chromium in the solution is between 0 ppm by wt. and 50 ppm by wt., and where the proportion of nickel in the solution is between 0 ppm by wt. and 50 ppm by wt.;
d) impregnating the support material with the solution;
e) drying the impregnated support material at temperatures between 50°C and 150°C;
f) calcining the dried, impregnated support material at temperatures between 300°C and 600°C to obtain a precursor;
g) activating the precursor by reduction with hydrogen to obtain the active catalyst,
**characterized in that** the impregnation and at least part of the drying is effected in a drum, in such a way that the support material is introduced into the drum for impregnation, that the drum is rotated, that the solution is sprayed into the drum, and that, during the drying phase, an air stream at a temperature between 50°C and 100°C is passed through the drum.

10. Process according to Claim 9, **characterized in that** the copper content of the solution is between 10.5% by wt. and 11.5% by wt.

11. Process according to either of Claims 9 and 10, **characterized in that** the support material provided comprises cylindrical extrudates having a diameter between 1 mm and 2 mm.

12. Process according to any of Claims 9 to 11, **characterized in that** the precursor has the following composition that adds up to 100% by wt.:
| | |
|---|---|
| silicon dioxide: | from 84% by wt. to 86% by wt.; |
| copper oxide: | from 14% by wt. to 16% by wt.; |
| nickel: | from 0 ppm by wt. to 50 ppm by wt.; |
| chromium: | from 0 ppm by wt. to 50 ppm by wt.; |
| aluminium oxide: | from 0 ppm by wt. to 1000 ppm by wt.; |
| other substances: | from 0 ppm by wt. to 1% by wt. |

13. Use of the catalysts prepared according to any of Claims 9 to 12 in processes for preparing alcohols by hydrogenation of aldehydes according to one or more of Claims 1 to 8.

## Revendications

1. Procédé pour la préparation d'alcools par l'hydrogénation d'aldéhydes, dans lequel au moins un aldéhyde ainsi qu'au moins un mélange d'alimentation contenant un composant associé sont mis en contact avec un catalyseur hétérogène en présence d'hydrogène, un mélange de produits étant obtenu, qui contient au moins l'alcool correspondant à l'aldéhyde hydrogéné ainsi qu'au moins un sous-produit, le catalyseur comprenant un matériau de support ainsi que du cuivre déposé sur celui-ci,
**caractérisé en ce que** le matériau de support est du dioxyde de silicium ;
et que le catalyseur sous forme activée présente la composition suivante, qui s'étend jusqu'à 100 % en poids :
dioxyde de silicium : de 86 % en poids à 90 % en poids ;
cuivre : de 10 % en poids à 14 % en poids ;
nickel : de 0 ppm en poids à 50 ppm en poids ;
chrome : de 0 ppm en poids à 50 ppm en poids ;
oxyde d'aluminium : de 0 ppm en poids à 1 000 ppm en poids ;
autres matières : de 0 ppm en poids à 1 % en poids.

2. Procédé selon la revendication 1, **caractérisé en ce que** le catalyseur sous forme activée présente la composition suivante, qui s'étend jusqu'à 100 % en poids :
dioxyde de silicium : de 87 % en poids à 89 % en poids ;
cuivre : de 11 % en poids à 13 % en poids ;
nickel : de 0 ppm en poids à 50 ppm en poids ;
chrome : de 0 ppm en poids à 50 ppm en poids ;
ruthénium : de 0 ppm en poids à 50 ppm en poids ;
palladium : de 0 ppm en poids à 50 ppm en poids ;
platine : de 0 ppm en poids à 50 ppm en poids ;
oxyde d'aluminium : de 0 ppm en poids à 100 ppm en poids ;
eau : de 0 ppm en poids à 100 ppm en poids ;
carbonates : de 0 ppm en poids à 100 ppm en poids ;
hydroxydes : de 0 ppm en poids à 100 ppm en poids ;
autres matières : de 0 ppm en poids à 0,5 % en poids.

3. Procédé selon la revendication 1 ou 2, **caractérisé en ce que** le volume spécifique de pore du matériau de support est compris entre 0,8 cm³/g et 1,2 cm³/g, déterminé selon la méthode d'immersion au cyclohexane, et **en ce que** la surface spécifique du matériau de support (surface BET) est comprise entre 130 m²/g et 170 m²/g, déterminée selon le procédé de la norme ISO 9277.

4. Procédé selon la revendication 1 ou selon l'une quelconque des revendications 2 et 3, **caractérisé en ce qu'**il est mis en œuvre à une pression comprise entre 15^{∗}10⁵ Pa et 25^{∗}10⁵ Pa et à une température comprise entre 140 °C et 180 °C, la pression et la température étant choisies de sorte que le mélange d'alimentation et le mélange de produits sont présents en une phase liquide.

5. Procédé selon la revendication 4, **caractérisé en ce que** l'hydrogène est présent en des quantités sub-stœchiométriques, la concentration de l'hydrogène étant choisie de telle façon qu'au moins une partie de l'hydrogène est présent dissous dans la phase liquide.

6. Procédé selon la revendication 1 ou selon l'une quelconque des revendications 2 à 5, **caractérisé en ce que** le mélange d'alimentation provient d'une hydroformylation et contient ainsi plusieurs aldéhydes dotés du même en nombre n d'atomes de carbone ainsi que les alcools correspondants et des composés à point d'ébullition élevé, n étant un nombre naturel compris entre 3 et 18.

7. Procédé selon la revendication 6, **caractérisé en ce que** le mélange d'alimentation présente la composition suivante, qui s'étend jusqu'à 100 % en poids :
proportion totale des aldéhydes comportant neuf atomes de carbone : 25 % en poids à 75 % en poids ;
proportion totale des alcools comportant neuf atomes de carbone : 10 % en poids à 55 % en poids ;
proportion totale d'acétals : 0,5 % en poids à 5,5 % en poids ;
proportion totale d'autres hydrocarbures : 0 % en poids à 40 % en poids ;
eau : 0 % en poids à 3 % en poids.

8. Procédé selon la revendication 6, **caractérisé en ce que** le mélange d'alimentation présente la composition suivante, qui s'étend jusqu'à 100 % en poids :
proportion totale des aldéhydes comportant neuf atomes de carbone : 15 % en poids à 65 % en poids ;
proportion totale des alcools comportant neuf atomes de carbone : 20 % en poids à 65 % en poids ;
proportion totale d'acétals : 0,5 % en poids à 5,5 % en poids ;
proportion totale d'autres hydrocarbures : 0 % en poids à 40 % en poids ;
eau : 0 % en poids à 1 % en poids.

9. Procédé pour la préparation d'un catalyseur comportant les étapes suivantes :
a) mise à disposition d'un matériau de support, qui contient au moins 99 % en poids de dioxyde de silicium ;
b) mise à disposition d'hydroxyde-carbonate de cuivre(II), d'hydrogénocarbonate d'ammonium et/ou de carbonate d'ammonium, d'ammoniaque et d'eau ;
c) préparation d'une solution d'hydroxyde-carbonate de cuivre(II), d'hydrogénocarbonate d'ammonium et/ou de carbonate d'ammonium, d'ammoniaque et d'eau, de sorte que la proportion de cuivre dans la solution est comprise entre 10 % en poids et 15 % en poids, la proportion en chrome dans la solution étant comprise entre 0 ppm en poids et 50 ppm en poids, et la proportion en nickel dans la solution étant comprise entre 0 ppm en poids et 50 ppm en poids ;
d) imprégnation du matériau de support avec la solution ;
e) séchage du matériau de support imprégné à des températures comprises entre 50 °C et 150 °C ;
f) calcination du matériau de support imprégné séché à des températures comprises entre 300 °C et 600 °C avec obtention d'un précurseur ;
g) activation du précurseur par réduction avec de l'hydrogène avec obtention du catalyseur activé,
**caractérisé en ce que** l'imprégnation et au moins une partie du séchage est réalisée dans un tambour, de sorte que le matériau de support est introduit dans le tambour pour imprégnation, que le tambour est mis en rotation, que la solution est injectée dans le tambour, et que pendant le séchage un flux d'air doté d'une température comprise entre 50 °C et 100 °C est passé dans le tambour.

10. Procédé selon la revendication 9, **caractérisé en ce que** la teneur en cuivre de la solution est comprise entre 10,5 % en poids et 11,5 % en poids.

11. Procédé selon l'une quelconque des revendications 9 et 10, **caractérisé en ce que** les matériaux de support mis à disposition sont des extrudats cylindriques dotés d'un diamètre compris entre 1 mm et 2 mm.

12. Procédé selon l'une quelconque des revendications 9 à 11, **caractérisé en ce que** le précurseur présente la composition suivante, qui s'étend jusqu'à 100 % en poids :
dioxyde de silicium : de 84 % en poids à 86 % en poids ;
oxyde de cuivre : de 14 % en poids à 16 % en poids ;
nickel : de 0 ppm en poids à 50 ppm en poids ;
chrome : de 0 ppm en poids à 50 ppm en poids ;
oxyde d'aluminium : de 0 ppm en poids à 1 000 ppm en poids ;
autres matières : de 0 ppm en poids à 1 % en poids.

13. Utilisation des catalyseurs préparés selon l'une quelconque des revendications 9 à 12 dans un procédé pour la préparation d'alcools par hydrogénation d'aldéhydes selon l'une ou plusieurs des revendications 1 à 8.
